(19) Europäisches Patentamt
European Patent Office
Office européen des brevets

(11) Veröffentlichungsnummer : **0 051 805**
**B1**

(12) # EUROPÄISCHE PATENTSCHRIFT

(45) Veröffentlichungstag der Patentschrift : **18.01.84**

(51) Int. Cl.³ : **C 07 C 85/11**, C 07 C 87/48

(21) Anmeldenummer : **81109101.6**

(22) Anmeldetag : **28.10.81**

(54) **Verfahren zur Herstellung von aromatischen Aminen.**

(30) Priorität : **06.11.80 DE 3041836**

(43) Veröffentlichungstag der Anmeldung : **19.05.82 Patentblatt 82/20**

(45) Bekanntmachung des Hinweises auf die Patenterteilung : **18.01.84 Patentblatt 84/03**

(84) Benannte Vertragsstaaten : **CH DE FR GB LI**

(56) Entgegenhaltungen :
**DE-A- 2 331 878**
**DE-A- 2 654 852**
**BERICHTE DER DEUTSCHEN CHEMISCHEN GESELLSCHAFT, Band 56, Nr. 10, 1923 Leipzig, Berlin K.W. ROSENMUND et al. "Über die katalytische Reduktion mehrfacher Kohlenstoff-Stickstoff-Bindungen" Seiten 2258 bis 2262**

(73) Patentinhaber : **HOECHST AKTIENGESELLSCHAFT**
**Postfach 80 03 20**
**D-6230 Frankfurt am Main 80 (DE)**

(72) Erfinder : **Berthold, Rüdiger, Dr.**
**Geierfeld 55**
**D-6232 Bad Soden am Taunus (DE)**
Erfinder : **Müller, Werner Heinrich, Dr.**
**Taunusblick 5**
**D-6239 Eppstein/Taunus (DE)**

**EP 0 051 805 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von aromatischen Aminen

Es ist bekannt, daß aus 2-Cyclohexenonoximen mit wasserabspaltenden Mitteln primäre aromatische Amine hergestellt werden können (DE-PS 26 54 852). In J. Amer. Chem. Soc. 69 (1947) 1907-1908 ist beschrieben, daß man aus 2-Cyclohexenon-azinen die entsprechenden primären aromatischen Amine herstellen kann, wenn man die Azine in Alkylaromaten, insbesondere Triethylbenzol, mit Edelmetallkatalysatoren erhitzt. Die Übertragung dieser Reaktion auf 2-Cyclohexenon-oxime scheiterte jedoch.

Überraschenderweise wurde nun gefunden, daß man aromatische Amine aus den entsprechenden cycloaliphatischen Oximen herstellen kann, wenn man das Oxim in einem ethergruppenhaltigen Lösemittel mit einem ein Edelmetall der 8. Nebengruppe des Periodensystems enthaltenden Katalysator auf Temperaturen von 150 bis 350 °C erhitzt. Sofern man hierbei von einem Cyclohexenonoxim ausgeht, wird bei dem erfindungsgemäßen Verfahren ein Mol Wasser abgespalten ; geht man von einem gesättigten Oxim aus, so findet zusätzlich eine katalytische Dehydrierung statt.

Im folgenden werden bevorzugte Ausgestaltungen der Erfindung näher erläutert.

Als Ausgangsmaterialien kommen sowohl gesättigte als auch ungesättigte ein- und mehrkernige Oxime in Betracht, die inerte Substituenten tragen können. Selbstverständlich müssen diese Substituenten so angeordnet sein, daß eine Aromatisierung des Moleküls möglich ist. Ausgeschlossen sind auch « sperrige » Substituenten in den zur Oximgruppe benachbarten Stellungen, da hierdurch der Kontakt zwischen dem Oximmolekül und dem Katalysator be- bzw. verhindert würde.

Bevorzugte Ausgangsmaterialien sind Cyclohex-2-en-1-onoxime bzw. die entsprechenden Tetrahydronaphthalin-1-on-oxime, da diese sehr selektiv zu den entsprechenden primären aromatischen Aminen führen. Besonders bevorzugt sind Cyclohex-2-en-1-on-oxime der Formel

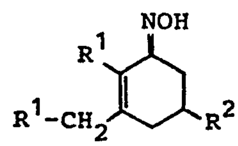

in der $R^1$ Wasserstoff, niederes Alkyl oder Phenyl und $R^2$ Wasserstoff, niederes Alkyl oder Phenyl bedeuten. Die zugrundeliegenden Ketone sind leicht aus den entsprechenden Acylessigestern und Aldehyden zugänglich, beispielsweise nach dem in der DE-OS 26 54 850 beschriebenen Verfahren. Aus diesen Ketonen sind die Oxime leicht zugänglich, beispielsweise nach dem in der DE-OS 26 54 851 beschriebenen Verfahren.

Da es sich bei dem erfindungsgemäßen Verfahren um eine Reaktion im heterogenen System handelt, ist es zweckmäßig, ständig für eine gute Durchmischung zu sorgen. Diese wird durch entsprechendes Rühren oder einfacher durch heftiges Sieden erreicht.

Bevorzugte Reaktionstemperaturen liegen zwischen 180 und 260 °C, da in diesem Bereich die Reaktion rasch und im allgemeinen mit hoher Selektivität abläuft. Je nach Art des eingesetzten Lösemittels wird bei Drücken von 0,001 bis 100, insbesondere von 0,01 bis 20 bar gearbeitet, wobei Druck und Temperatur zweckmäßig so aufeinander abgestimmt sind, daß genügend flüssige Phase vorhanden ist und die erwünschte Reaktionstemperatur aufrechterhalten wird. Als Lösemittel eignen sich grundsätzlich alle Ether, bevorzugt sind aliphatische Ether, also solche Ether, die mindestens eine aliphatische Gruppe enthalten. Vorzugsweise werden Ether mit einem Siedepunkt von mindestens etwa 80 °C eingesetzt, da bei niedriger siedenden Ethern relativ hohe Drücke erforderlich sind, um eine genügend hohe Reaktionstemperatur zu erreichen, bei der die Reaktion in annehmbarer Zeit zu Ende geführt werden kann.

Geeignet sind höher siedende Dialkylether, beispielsweise Di-n-butylether, Alkyl-aryl-ether, wie Anisol und Phenetol, insbesondere jedoch Polyether, die sich vom Ethylen- und/oder Propylenglykol ableiten. Als solche Polyether kommen im einfachsten Fall die niederen Dialkylether des Ethylenglykols und Propylenglykols in Betracht, vorzugsweise jedoch niedere Dialkylether von Polyglykolen wie Ethylendi-, -tri- oder -tetraglykol oder von höheren Polylglykolen, weiterhin auch Monoarylether wie Anlagerungsprodukte von Ethylenoxid an Alkylphenole wie Nonylphenol oder Tributylphenol, deren freie Hydroxygruppe mit niederen Alkanolen verethert sein kann. Als Ether kommen jedoch auch höhere Polyglykole mit freien Hydroxy-Endgruppen in Betracht, bevorzugt sind jedoch ihre niederen Dialkylether mit bis zu 6 Kohlenstoffatomen in den Ethergruppen, insbesondere die Methyl- und Ethylether.

Zweckmäßig wählt man einen Ether, der bei atmosphärischem Druck im bevorzugten Temperaturbereich zwischen 180 und 260 °C siedet, da mit diesem Lösemittel drucklos gearbeitet werden kann und die Reaktion unter Rückflußbedingungen besonders rasch und selektiv verläuft. Hierbei kann — abhängig von den Reaktionsbedingungen — der Siedepunkt des Lösemittels so gewählt werden, daß sich das Amin bei der Aufarbeitung im Destillationssumpf oder im Destillat findet. Eine zweckmäßige Ausgestaltung der Erfindung besteht auch darin, daß man einen hochsiedenden Ether einsetzt und das entstandene Amin in dem Maße, wie es entsteht, abdestilliert, gegebenenfalls unter vermindertem Druck. Auf diese Weise kommt man mit einer geringen Menge an Lösemittel aus. Wird ein Ether eingesetzt, der tiefer als das

entstehende Amin siedet, so kann man das Lösemittel laufend abdestillieren und — um Lösemittel einzusparen — in den Prozeß zurückführen, wobei das Amin aus dem Sumpf ausgeschleust wird. Diese Verfahren lassen sich auch vollkontinuierlich ausgestalten. Hierbei wird beispielsweise eine Lösung des Oxims in demselben Lösemittel, das auch zur Suspendierung des Katalysators verwendet wird, oder das geschmolzene Oxim kontinuierlich über einen Vorheizer in den Reaktor eingebracht, während gleichzeitig eine entsprechende Menge der Reaktionsmischung, die das gebildete Amin enthält, ausgetragen wird. Der Katalysator wird hierbei beispielsweise mittels einer Fritte im Reaktor zurückgehalten oder nach Abtrennung, z. B. mittels eines Dekanters, in den Reaktor zurückgeführt. Das Lösemittel wird nach destillativer Abtrennung vom gebildeten Amin wieder zum Lösen von neuem Oxim verwendet.

Es ist auch möglich, das Oxim in einem tiefsiedenden Lösemittel gelöst zuzugeben, das während der Reaktion laufend abdestilliert. Als solches tiefsiedendes Lösemittel für das Oxim kommen nicht nur Ether, sondern auch andere hinreichend inerte Lösemittel in Betracht, beispielsweise niedere Alkanole wie Isopropanol.

Neben der Art des Lösemittels hat auch die Menge einen gewissen Einfluß auf die Reaktion, da mit steigender Konzentration des eingesetzten Oxims und des gebildeten Amins die Ausbeute abnimmt. Grundsätzlich sind die Ausbeuten um so höher, je niedriger die Konzentration ist. Aus wirtschaftlichen Gründen wählt man eine Oxim- und Aminkonzentration im Reaktionsgemisch von bis zu etwa 30 Gew.-%, vorzugsweise bis zu etwa 15 %, bezogen auf das Gewicht des Lösemittels.

Um die Oximkonzentration möglichst niedrig zu halten, stellt man zweckmäßig die Geschwindigkeit der Oximzugabe auf die Durchsatz-Kapazität des eingesetzten Katalysators ein, was durch einen einfachen Vorversuch leicht zu ermitteln ist.

Die Katalysatoren sind entweder Skelett- oder Trägerkatalysatoren, die als wirksames Metall zweckmäßig Ruthenium, Rhodium, Iridium, insbesondere jedoch Palladium oder Platin oder mehrere dieser Metalle enthalten. Als Träger für die Katalysatoren kommen üblicherweise Kohle, Siliziumdioxid, Aluminiumoxid, Aluminiumsilikate, Mischoxide wie Chromoxid-Aluminiumoxid oder Spinelle, Bariumsulfat oder mineralische Träger wie Zeolithe in Betracht. Die Konzentration des Edelmetalls auf dem Träger beträgt im allgemeinen 0,05 bis 10, vorzugsweise 0,2 bis 5, insbesondere 0,5 bis 2,5 %, bezogen auf das Gewicht des Trägers.

Wenn die handelsüblichen Trägerkatalysatoren eingesetzt werden, wählt man eine Katalysator-Teilchengröße von etwa 0,01 bis 5 mm, vorzugsweise 0,05 bis 1 mm. Abhängig vom Lösemittel und vom Katalysator kann die Reaktions-Suspension 0,1 bis 40 % Trägerkatalysator, bezogen auf das Gewicht des flüssigen Reaktionsmediums, enthalten. Bevorzugt sind 1 bis 30 %, bezogen auf das Gewicht des Lösemittels.

Die Aktivität des Katalysators nimmt im Laufe der Reaktion allmählich ab, wobei im gleichen Maße Nebenprodukte gebildet werden. Diese Nebenprodukte bleiben bei der destillativen Abtrennung des Amins im Rückstand. Zur Abtrennung vom Lösemittel kann der vom Katalysator abgesaugte Destillationsrückstand auf Wasser gegossen werden, worauf die ungelösten Nebenprodukte abgetrennt und das mit Kohle geklärte wäßrige Filtrat durch Destillation entwässert wird.

In den folgenden Beispielen beziehen sich Prozentangaben auf das Gewicht, sofern nichts anderes angegeben ist.

Beispiel 1

In einen 250 ml Vierhalskolben, der mit einer kleinen Vigreux-Kolonne und einem absteigenden Kühler versehen ist, tropft man unter gutem Rühren innerhalb von 4 Stunden eine Lösung von 12 g 3-Methyl-5-propyl-2-cyclohexenonoxim (MG 167) in 100 ml Isopropanol zu einer Suspension von 10 g eines Katalysators, bestehend aus 5 % Pd auf $Al_2O_3$-Pulver, in 100 ml Methylbutyl-diethylenglykol (Kp. 215 °C). Die Reaktionsmischung wird auf eine Innentemperatur von 210 °C erhitzt und das Isopropanol gleichmäßig über die Kolonne abdestilliert. Wenn alles Oxim zugetropft ist, rührt man noch 30 Minuten nach und saugt dann die erkaltete Lösung vom Katalysator ab. Man erhält 85,5 g Reaktionslösung. Die gaschromatographische Analyse ergab einen Gehalt von 11,3 % 3-Propyl-5-methylanilin (MG 149), entsprechend 9,66 g = 90,0 % d. Th., sowie 0,6 % (entspr. 0,51 g = 5,0 % d. Th.) des Diarylamins der Formel

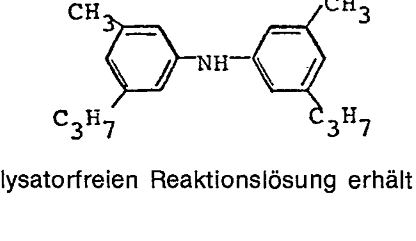

(MG 281)

Durch Fraktionieren der katalysatorfreien Reaktionslösung erhält man 3-Propyl-5-methyl-anilin ; Kp 106 °C bei 1,2 mbar.

Beispiel 2

In der in Beispiel 1 beschriebenen Weise wurden 15 g (108 mmol) 3,5-Dimethyl-2-cyclohexenonoxim,

gelöst in 100 ml Isopropanol, mit einem Pd-Katalysator (5 % Pd auf Kohle, Firma Engelhardt) und Diethyl-diethylenglykol (Kp 178 °C) als Lösemittel bei 175-180 °C umgesetzt.

Man erhält 81 g Lösung mit einem gaschromatographisch ermittelten Xylidingehalt von 15 %. Daraus ergibt sich die Ausbeute zu 12,15 g sym. m-Xylidin, entsprechend 93 % d. Th. Daneben fallen 0,6 g Dixylylamin (MG 225) entsprechend 4,9 % d. Th. an. Durch fraktionierte Destillation erhält man sym. m-Xylidin vom Kp. 226-228 °C.

Entsprechend erhält man aus 2-Ethyl-3-propyl-5-methyl-2-cyclohexenonoxim 3-Methyl-5-propyl-6-ethyl-anilin in 89 % Ausbeute, aus Tetrahydronaphthalin-1-oxim 1-Naphthylamin in 56 % Ausbeute und aus 3-Methyl-5-phenyl-2-cyclohexenonoxim 3-Methyl-5-phenyl-anilin in 84 % Ausbeute.

Beispiel 3

11,3 g Cyclohexanon-oxim (MG 113), gelöst in 100 ml Isopropanol, tropft man zu einer Suspension eines Pd-$Al_2O_3$-Katalysators in 100 ml Diethyl-diethylenglykol, wie in Beispiel 1 angegeben. Man erhält 71,3 g Lösung mit einem Gehalt von 1,7 % Anilin (= 1,2 g, entsprechend 13 % d. Th.) und 7,8 % Diphenylamin (MG 169) (= 5,6 g, entspr. 65,8 % d. Th.).

Beispiel 4

In einen 1,5 l Edelstahlreaktor, ausgerüstet mit Rührer, Rückflußkühler und automatischer Stand- und Druckhaltung, werden 50 g eines 2,8 % Pd-$Al_2O_3$-Pulver-Katalysators und 1 l Dimethyl-diethylenglykol gegeben. Unter Stickstoffspülung wird aufgeheizt. Die automatische Druckhaltung wird auf 1,6 bar eingestellt, wobei sich eine Rückflußtemperatur von 190 °C einstellt. Nun werden stündlich 100 g 3,5-Dimethyl-2-cyclohexenon-oxim, gelöst in 900 g Dimethyldiethylenglykol, zugepumpt und gleichzeitig aus dem Reaktor 1 000 g Reaktionsprodukt über eine Filtervorrichtung, die den Katalysator im Reaktor zurückhält, abgenommen. Die gaschromatographische Analyse des Reaktionsprodukts zeigt 7,93 % sym. m-Xylidin (91 % d. Th.). Nach Abdestillieren des Dimethyldiethylenglykols destillierte bei Kp 93 °C/12 mbar 99,6 %iges Xylidin über.

**Ansprüche**

1. Verfahren zur Herstellung von aromatischen Aminen aus den entsprechenden cycloaliphatischen Oximen, dadurch gekennzeichnet, daß man das Oxim in einem ethergruppenhaltigen Lösemittel mit einem ein Edelmetall der 8. Nebengruppe des Periodensystems enthaltenden Katalysator auf Temperaturen von 150 bis 350 °C erhitzt.

2. Verfahren nach Anspruch 1, dadurch gekennzeichnet, daß das Oxim ein Cyclohex-2-en-1-onoxim oder ein Tetrahydronaphthalin-1-onoxim ist.

3. Verfahren nach Anspruch 1, dadurch gekennzeichnet, daß das Oxim ein Cyclohex-2-en-1-on-oxim der Formel

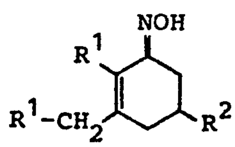

ist, in der $R^1$ Wasserstoff, niederes Alkyl oder Phenyl und $R^2$ Wasserstoff, niederes Alkyl oder Phenyl bedeuten.

4. Verfahren nach Anspruch 1 bis 3, dadurch gekennzeichnet, daß das ethergruppenhaltige Lösemittel ein aliphatischer Ether ist.

5. Verfahren nach Anspruch 1 bis 4, dadurch gekennzeichnet, daß das ethergruppenhaltige Lösemittel ein niederer Dialkylether eines Polyethylenglykols ist.

6. Verfahren nach Anspruch 1 bis 5, dadurch gekennzeichnet, daß die Konzentration des Oxims im Reaktionsgemisch 30 Gewichtsprozent nicht überschreitet.

7. Verfahren nach Anspruch 1 bis 6, dadurch gekennzeichnet, daß die Konzentration des Oxims 15 Gewichtsprozent, bezogen auf das Lösemittel, nicht überschreitet.

8. Verfahren nach Anspruch 1 bis 7, dadurch gekennzeichnet, daß man das Oxim zum erhitzten Lösemittel zugibt.

**Claims**

1. Process for the preparation of aromatic amines from the corresponding cycloaliphatic oximes, which comprises heating the oxime to a temperature of from 150 to 350 °C in an ether group containing

solvent with a catalyst containing a noble metal of the 8th auxiliary group of the Periodic Table of Elements.

2. Process as claimed in Claim 1, wherein the oxime is a cyclohex-2-en-1-one oxime or a tetrahydronaphthalen-1-one oxime.

3. Process as claimed in Claim 1, wherein the oxime is a cyclohex-2-ene-1-on-oxime of the formula

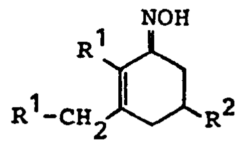

in which $R^1$ and $R^2$ each represent hydrogen, lower alkyl or phenyl.

4. Process as claimed in Claims 1 to 3, wherein the ether group containing solvent is an aliphatic ether.

5. Process as claimed in Claims 1 to 4, wherein the ether group containing solvent is a lower dialkyl ether of a polyethyleneglycol.

6. Process as claimed in Claims 1 to 5, wherein the concentration of the oxime in the reaction mixture does not exceed 30 weight %.

7. Process as claimed in Claims 1 to 6, wherein the concentration of the oxime does not exceed 15 weight %, referred to the solvent.

8. Process as claimed in Claims 1 to 7, wherein the oxime is added to the heated solvent.

**Revendications**

1. Procédé de préparation d'amines aromatiques à partir des oximes cycloaliphatiques correspondantes, procédé caractérisé en ce qu'on chauffe l'oxime à des températures de 150 à 350 °C, dans un solvant contenant des radicaux d'éther, avec un catalyseur contenant un métal noble du huitième sous-groupe de la classification périodique.

2. Procédé selon la revendication 1, caractérisé en ce que l'oxime est une cyclohexène-2 one-1 oxime ou une tétrahydronaphtalène-one-1 oxime.

3. Procédé selon la revendication 1, caractérisé en ce que l'oxime est une cyclohexène-2 one-1 oxime de formule :

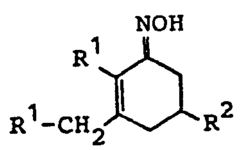

dans laquelle $R^1$ représente l'hydrogène, un alkyle inférieur ou un phényle et $R^2$ représente l'hydrogène, un alkyle inférieur ou un phényle.

4. Procédé selon l'une quelconque des revendications 1 à 3, caractérisé en ce que le solvant contenant des radicaux d'éther est un éther aliphatique.

5. Procédé selon l'une quelconque des revendications 1 à 4, caractérisé en ce que le solvant contenant des radicaux d'éther est un éther dialkylique inférieur d'un poéthylène-glycol.

6. Procédé selon l'une quelconque des revendications 1 à 5, caractérisé en ce que la concentration du mélange réactionnel en l'oxime ne dépasse pas 30 % en poids.

7. Procédé selon l'une quelconque des revendications 1 à 6, caractérisé en ce que la concentration en l'oxime ne dépasse pas 15 % en poids, par rapport au solvant.

8. Procédé selon l'une quelconque des revendications 1 à 7, caractérisé en ce qu'on ajoute l'oxime au solvant chauffé.